# EUROPEAN PATENT SPECIFICATION

(11) **EP 4 210 802 B1**
(45) Date of publication and mention of the grant of the patent: **14.10.2026**
(21) Application number: 21867402.6
(22) Date of filing: 03.09.2021
(51) Int. Cl.: A61M 25/00, A61M 25/01, A61M 25/06, A61M 25/10

(54) **SELF CENTERING LOW FRICTION EXTENDABLE NEUROVASCULAR CATHETER**
SELBSTZENTRIERENDER, REIBUNGSARMER, EXPANDIERBARER NEUROVASKULÄRER KATHETER
CATHÉTER NEUROVASCULAIRE EXTENSIBLE À FAIBLE FROTTEMENT À CENTRAGE AUTOMATIQUE

(30) Priority: 14.09.2020 US 202063078143 P
(43) Date of publication of application: 19.07.2023
(73) Proprietor: Imperative Care, Inc., Campbell, California 95008 (US)
(72) Inventor: LUNA, Michael, Campbell, California 95008 (US); ROUE, Chad C., Campbell, California 95008 (US); YANG, Yi, Campbell, California 95008 (US)
(74) Representative: ABG Intellectual Property Law, S.L.
(86) International application number: PCT/US2021/049066
(87) International publication number: WO 2022/055810

(56) References cited:
- CN-A- 108 135 591
- US-A- 5 916 192
- US-A1- 2017 143 938
- US-A1- 2020 008 820
- US-A1- 2020 008 820
- US-B2- 10 179 224
- US-B2- 7 112 298
- US-B2- 9 265 512

## Description

### CROSS-REFERENCE TO RELATED APPLICATIONS

This application claims the priority benefit of U.S. Provisional Patent Application No. 63/078,143, filed September 14, 2020.

### BACKGROUND

Stroke is the third most common cause of death in the United States and the most disabling neurologic disorder. Approximately 700,000 patients suffer from stroke annually. Stroke is a syndrome characterized by the acute onset of a neurological deficit that persists for at least 24 hours, reflecting focal involvement of the central nervous system, and is the result of a disturbance of the cerebral circulation. Its incidence increases with age. Risk factors for stroke include systolic or diastolic hypertension, hypercholesterolemia, cigarette smoking, heavy alcohol consumption, and oral contraceptive use.

Hemorrhagic stroke accounts for 20% of the annual stroke population. Hemorrhagic stroke often occurs due to rupture of an aneurysm or arteriovenous malformation bleeding into the brain tissue, resulting in cerebral infarction. The remaining 80% of the stroke population are ischemic strokes and are caused by occluded vessels that deprive the brain of oxygen-carrying blood. Ischemic strokes are often caused by emboli or pieces of thrombotic tissue that have dislodged from other body sites or from the cerebral vessels themselves to occlude in the narrow cerebral arteries more distally. When a patient presents with neurological symptoms and signs which resolve completely within 1 hour, the term transient ischemic attack (TIA) is used. Etiologically, TIA and stroke share the same pathophysiologic mechanisms and thus represent a continuum based on persistence of symptoms and extent of ischemic insult.

Emboli occasionally form around the valves of the heart or in the left atrial appendage during periods of irregular heart rhythm and then are dislodged and follow the blood flow into the distal regions of the body. Those emboli can pass to the brain and cause an embolic stroke. As will be discussed below, many such occlusions occur in the middle cerebral artery (MCA), although such is not the only site where emboli come to rest.

When a patient presents with neurological deficit, a diagnostic hypothesis for the cause of stroke can be generated based on the patient's history, a review of stroke risk factors, and a neurologic examination. If an ischemic event is suspected, a clinician can tentatively assess whether the patient has a cardiogenic source of emboli, large artery extracranial or intracranial disease, small artery intraparenchymal disease, or a hematologic or other systemic disorder. A head CT scan is often performed to determine whether the patient has suffered an ischemic or hemorrhagic insult. Blood would be present on the CT scan in subarachnoid hemorrhage, intraparenchymal hematoma, or intraventricular hemorrhage.

Traditionally, emergent management of acute ischemic stroke consisted mainly of general supportive care, e.g. hydration, monitoring neurological status, blood pressure control, and/or anti-platelet or anti-coagulation therapy. In 1996, the Food and Drug Administration approved the use of Genentech Inc.'s thrombolytic drug, tissue plasminogen activator (t-PA) or Activase^{®}, for treating acute stroke. A randomized, double-blind trial, the National Institute of Neurological Disorders and t-PA Stroke Study, revealed a statistically significant improvement in stoke scale scores at 24 hours in the group of patients receiving intravenous t-PA within 3 hours of the onset of an ischemic stroke. Since the approval of t-PA, an emergency room physician could, for the first time, offer a stroke patient an effective treatment besides supportive care.

However, treatment with systemic t-PA is associated with increased risk of intracerebral hemorrhage and other hemorrhagic complications. Patients treated with t-PA were more likely to sustain a symptomatic intracerebral hemorrhage during the first 36 hours of treatment. The frequency of symptomatic hemorrhage increases when t-PA is administered beyond 3 hours from the onset of a stroke. Besides the time constraint in using t-PA in acute ischemic stroke, other contraindications include the following: if the patient has had a previous stroke or serious head trauma in the preceding 3 months, if the patient has a systolic blood pressure above 24.665 kPa (185 mm Hg) or diastolic blood pressure above 14.665 kPa (110 mm Hg), if the patient requires aggressive treatment to reduce the blood pressure to the specified limits, if the patient is taking anticoagulants or has a propensity to hemorrhage, and/or if the patient has had a recent invasive surgical procedure. Therefore, only a small percentage of selected stroke patients are qualified to receive t-PA.

Obstructive emboli have also been mechanically removed from various sites in the vasculature for years. Mechanical therapies have involved capturing and removing the clot, dissolving the clot, disrupting and suctioning the clot, and/or creating a flow channel through the clot. One of the first mechanical devices developed for stroke treatment is the MERCI Retriever System (Concentric Medical, Redwood City, Calif.). A balloon-tipped guide catheter is used to access the internal carotid artery (ICA) from the femoral artery. A microcatheter is placed through the guide catheter and used to deliver the coil-tipped retriever across the clot and is then pulled back to deploy the retriever around the clot. The microcatheter and retriever are then pulled back, with the goal of pulling the clot, into the balloon guide catheter while the balloon is inflated and a syringe is connected to the balloon guide catheter to aspirate the guide catheter during clot retrieval. This device has had initially positive results as compared to thrombolytic therapy alone.

Other thrombectomy devices utilize expandable cages, baskets, or snares to capture and retrieve clot. Temporary stents, sometimes referred to as stentrievers or revascularization devices, are utilized to remove or retrieve clot as well as restore flow to the vessel. A series of devices using active laser or ultrasound energy to break up the clot have also been utilized. Other active energy devices have been used in conjunction with intra-arterial thrombolytic infusion to accelerate the dissolution of the thrombus. Many of these devices are used in conjunction with aspiration to aid in the removal of the clot and reduce the risk of emboli. Suctioning of the clot has also been used with single-lumen catheters and syringes or aspiration pumps, with or without adjunct disruption of the clot. Devices which apply powered fluid vortices in combination with suction have been utilized to improve the efficacy of this method of thrombectomy. Finally, balloons or stents have been used to create a patent lumen through the clot when clot removal or dissolution was not possible.

Notwithstanding the foregoing, there remains a need for new devices and methods for treating vasculature occlusions in the body, including acute ischemic stroke and occlusive cerebrovascular disease.

Document US2020/0008820A1 discloses a sealed neurovascular extendable catheter.

### SUMMARY

The invention provides an extendable neurovascular catheter according to claim 1.

Disclosed herein is an extendable neurovascular catheter having an outer catheter, an inner extension segment, and an annular gap between an outer surface of the inner extension segment and an inner surface of the outer catheter. The extendable catheter also has at least one displacement or centering feature positioned between the inner extension segment and the outer catheter. The centering feature is configured to laterally displace the inner extension segment from an adjacent sidewall of the outer catheter, or to center the inner extension segment by only partially filling the gap to reduce total contact surface area between the extension segment and outer catheter and allow axial translation of the extension segment relative to the outer catheter with minimal friction. This results in a flow path between adjacent projections and the outer catheter and inner extension segment.

There is provided in accordance with one aspect of the invention an extendable neurovascular catheter system. The system comprises an outer catheter having a proximal end, a distal end, a sidewall defining a central lumen, and a longitudinal axis, and an inner extension segment having a proximal end and a distal end and extendable through the central lumen such that the distal end of the extension segment is configured to extend beyond the distal end of the outer catheter. The extension segment and central lumen are dimensioned to provide an annular gap between an outer surface of the extension segment and an inner surface of the central lumen. A discontinuous plurality of projections are positioned between the extension section and the outer catheter and configured to center at least a portion of a length of the extension segment within the central lumen, and to maintain a preset distance between the extension section and the sidewall.

The extendable neurovascular catheter system includes at least three projections, and in some implementations at least about 10 or 20 or more carried in the annular gap. The projections may be carried on an outside surface of the extension section. The system may additionally include an axially movable control wire extending proximally through the outer catheter from the extension section. The system may additionally include at least one centering feature made from a plurality of projections arranged in a pattern, the centering feature extending circumferentially in a discontinuous configuration around the extension segment. The centering feature may be inclined at a non normal angle with respect to the longitudinal axis.

There is provided in accordance with another aspect of the invention an extension segment for an aspiration system. The aspiration system includes a primary catheter with a distal end and a central lumen for axially movably receiving the extension segment. The extension segment has a shorter axial length than the catheter. The extension segment comprises an elongate, flexible tubular body, having a proximal end, a distal end, an outside surface and a central lumen. A control wire extends proximally from the proximal end and is configured for advancing the distal end of the extension beyond the distal end of the primary catheter. A plurality of projections are provided on the outside surface of the extension section. The projections maintain a minimum space between the outside surface of the extension section and a surface defining the central lumen. In one implementation, the projections comprise a plurality of axially extending ridges.

Any feature, structure, or step disclosed herein can be replaced with or combined with any other feature, structure, or step disclosed herein, or omitted. Further, for purposes of summarizing the disclosure, certain aspects, advantages, and features of the embodiments have been described herein. It is to be understood that not necessarily any or all such advantages are achieved in accordance with any particular embodiment disclosed herein. No individual aspects of this disclosure are essential or indispensable. Further features and advantages of the embodiments will become apparent to those of skill in the art in view of the Detailed Description which follows when considered together with the attached drawings and claims. In the description certain references are made to non-SI units (inches and pounds). These are to be translated to corresponding SI units (mm and kg) using the factor: 1 inch = approximately 25.4 mm and 1 pound = 0.454 kg.

### BRIEF DESCRIPTION OF THE DRAWINGS

FIG. 1 is a side elevational schematic view of an intracranial aspiration catheter in accordance with the present invention, with a distal extension segment in a proximally retracted configuration.
FIG. 2 is a side elevational view as in FIG. 1, with the distal extension segment in a distally extended configuration.
FIGS. 3A-3B are cross-sectional elevational views of a distal end of catheter 10, with the distal extension segment 34 fully extended.
FIGS. 4A - 4B are side elevational views of two different distal extension segments.
FIG. 5A is a schematic perspective view of a distal extension segment with a plurality of sidewall projections.
FIGS. 5B -5D show alternative sidewall projection configurations.
FIG. 6 is a cross section taken along the line 6-6 in Figure 5.
FIG. 7 is a schematic perspective view of a distal extension segment with a plurality of sidewall projections of an alternative configuration.
FIGS. 8A - 8C show the profile of a centering feature having a chevron side view configuration, with the projections omitted for clarity.
FIGS. 9A - 9C show various projection configurations within the profile illustrated in Figures 8A - 8C.
FIG. 10 illustrates a cross-sectional detail view of a distal portion of a catheter sidewall.
FIG. 11A describes a side elevational view of the catheter of FIG. 10.
FIG. 11B illustrates a cross-sectional view taken along the line C-C of FIG. 11A, showing one or more axially extending filaments.
FIG. 11C is a side elevational cross section through an angled distal catheter or extension tube tip.
FIG. 12 shows a sidewall polymer jacket configuration useful for either the aspiration catheter or the extension segment.

### DETAILED DESCRIPTION

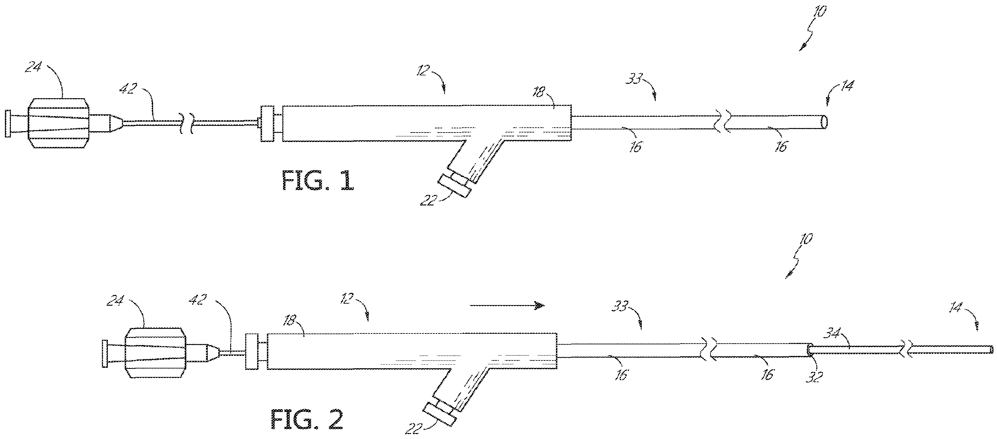

Referring to FIG. 1, there is disclosed a catheter 10 in accordance with one aspect of the present invention. Although primarily described in the context of an axially extendable distal segment aspiration catheter with a single central lumen, catheters of the present invention can readily be modified to incorporate additional structures, such as permanent or removable column strength enhancing mandrels, two or more lumen such as to permit drug, contrast or irrigant infusion or to supply inflation media to an inflatable balloon carried by the catheter, or combinations of these features, as will be readily apparent to one of skill in the art in view of the disclosure herein. In addition, the present invention will be described primarily in the context of removing obstructive material from remote vasculature in the brain, but has applicability as an access catheter for delivery and removal of any of a variety of diagnostics or therapeutic devices with or without aspiration.

The catheters disclosed herein may readily be adapted for use throughout the body wherever it may be desirable to distally advance a low profile distal catheter segment from a larger diameter proximal segment. For example, axially extendable catheter shafts in accordance with the present invention may be dimensioned for use throughout the coronary and peripheral vasculature, the gastrointestinal tract, the urethra, ureters, Fallopian tubes and other lumens and potential lumens, as well. The telescoping structure of the present invention may also be used to provide minimally invasive percutaneous tissue access, such as for diagnostic or therapeutic access to a solid tissue target (e.g., breast or liver or brain biopsy or tissue excision), delivery of laparoscopic tools or access to bones such as the spine for delivery of screws, bone cement or other tools or implants.

The catheter 10 generally comprises an elongate tubular body 16 extending between a proximal end 12 and a distal functional end 14. The length of the tubular body 16 depends upon the desired application. For example, lengths in the area of from about 120 cm to about 140 cm or more are typical for use in femoral access percutaneous transluminal coronary applications. Intracranial or other applications may call for a different catheter shaft length depending upon the vascular access site, as will be understood in the art.

In the illustrated embodiment, the tubular body 16 is divided into at least a fixed proximal section 33 and an axially extendable and retractable distal section 34 separated at a transition 32, which may be the distal end of the proximal section 33. Proximal and distal movement of the distal section 34 is controlled by a control wire 42, discussed further below. FIG. 2 is a side elevational view of the catheter 10 shown in FIG. 1, with the distal segment in a distally extended configuration.

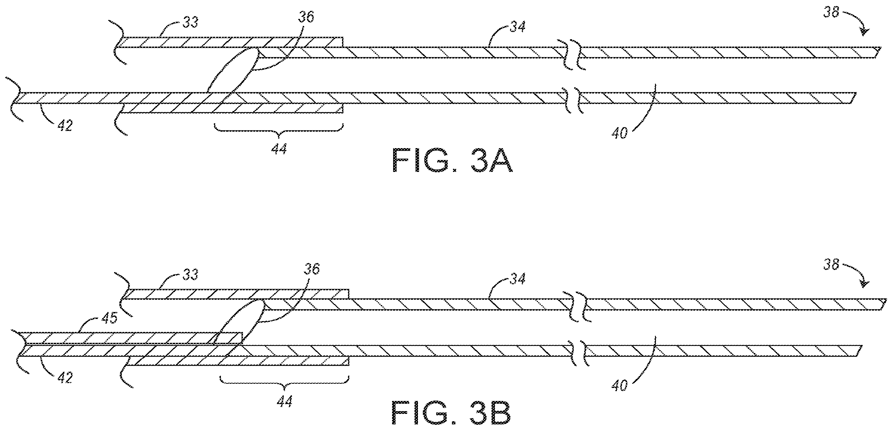

Referring to FIG. 3A and 3B, there is illustrated a cross-sectional view of the distal segment 34 shown extended distally from the proximal segment 33 in accordance with the present invention. Distal segment 34 extends between a proximal end 36 and a distal end 38 and defines at least one elongate central lumen 40 extending axially therethrough.

The inner diameter of the distal section 34 may be between about 0.030 inches and about 0.112 inches, between about 0.055 inches and about 0.087 inches, between about 0.064 inches and about 0.078 inches, or between about 0.068 inches and about 0.074 inches depending upon the intended clinical use.

The inner diameter and the outer diameter of the distal section 34 may be constant or substantially constant along its longitudinal length. The total thickness of the sidewall extending between the inner and outer diameter may be at least about 0.005 inches, 0.010 inches, 0.015 inches, 0.02 inches, but generally less than about 0.020 inches, or less than 0.015 inches. For example, the distal section may 34 may have an inner diameter of about 0.071 inches and an outer diameter of about 0.083 inches.

Alternatively, the distal section 34 may be tapered near its distal end. The distal section 34 may be tapered along a length within about 5cm, about 10cm, or about 20cm from its distal end.

In some embodiments, the proximal segment 33 may have an inner diameter of at least about 0.08 inches, 0.1 inches or more. The proximal segment 33 may have an outer diameter of at least about 0.05 inches, 0.10 inches or more, but generally less than about 0.020 0r about 0.015 inches. For example, the inner diameter may be approximately 0.088 inches and the outer diameter may be approximately 0.106 inches.

The length of the proximal segment 33 may be at least about 90 cm, 120 cm or more. For example, in one embodiment the length is approximately 106 cm. In another embodiment, the length is approximately 117 cm. In some neurovascular applications, the distal end of the proximal segment 33 may extend from a femoral artery access to at least to the Horizontal Petrous segment of the vasculature.

In some embodiments, the length of the distal section 34 may be between about 10 cm and about 50 cm, between about 15 cm and about 40 cm, between about 20 cm and 30 cm. The length of the distal section 34 may be less than or equal to about 20cm, about 25cm, about 30cm, or about 40 cm.

The proximal end 36 of distal section 34 is provided with a proximally extending control wire 42. Control wire 42 extends proximally throughout the length of the tubular body 16, to control 24 which may be a hub, a control on a proximal manifold or proximal portion of control wire 42. Axial movement of control 24 produces a corresponding axial movement of distal section 34 with respect to proximal section 33 as has been discussed. In some implementations the length of the control wire 42 may be between approximately 100 - 160 cm or between about 110-120 cm.

Upon distal advance of control wire 42 to its limit of travel, an overlap 44 remains between the proximal end 36 of distal section 34 and the proximal section 33. This overlap 44 is configured to prevent excessive distal advance of the extension segment and maintain efficient transmission of vacuum from proximal section 33 to distal section 34.

Following positioning of the distal end of proximal section 33 within the vasculature, such as within the cervical carotid artery, the control 24 is manipulated to distally advance distal section 34 deeper into the vasculature. For this purpose, the control wire 42 will be provided with sufficient column strength to enable distal advance of the distal tip 38 as will be discussed below.

The control wire 42 and distal section 34 may be integrated into a catheter. Alternatively, distal section 34 and control wire 42 may be configured as a stand-alone catheter extension device as is discussed in greater detail below. The catheter extension device may be introduced into the proximal end of a catheter such as proximal section 33 after placement of proximal section 33, and advanced distally there through, to telescopically extend the reach of the aspiration system.

Referring to FIG. 3A and 3B, the control wire 42 may comprise a solid core wire, or a tubular side wall having an axially extending central lumen 45. The centering feature discussed below has been omitted for clarity. The central lumen 45 permits introduction of media such as lubricants, drugs, contrast agents or others into the distal section 34. In addition, the central lumen 45 extending through control wire 42 permits introduction of an agitator as is discussed in US patent No. 10,183,145 to Yi, et al. and issued January 22, 2019.

As shown in FIG. 3B, the central lumen 45 may open into the lumen 40. The distal opening of the central lumen 45 may be positioned at a point along the length of the distal section 34 such that the central lumen 45 terminates where the lumen 40 begins (the distal opening of central lumen 45 may be longitudinally aligned with the proximal opening of lumen 40), or within about 2 or 5 or 10 cm of the proximal opening of lumen 40. The proximal opening of lumen 40 may be angled or slanted as shown in FIG. 3A and 3B.

The control wire 42 may be relatively stiff along the portion proximal to the proximal end of the distal section 34 in order to provide sufficient pushability of the extension catheter. The stiffness of the portion of the control wire 42 distal of the proximal end of the distal section 34 may substantially match or be less than the stiffness of the distal section 34 along the length of the distal section 34. The portion of the control wire 42 distal of the proximal end of the distal section 34 may have a uniform stiffness less than the stiffness of the portion proximal of the proximal end of the distal section 34 or it may have a gradated or gradually decreasing stiffness in the distal direction, decreasing from the stiffness of the portion proximal of the proximal end of the distal section 34.

For example, the control wire 42 may comprise metal (e.g., stainless steel or Nitinol hypotube) along the portion proximal to the proximal end of the distal section 34 and may comprise a polymer, softer than the metal, along the portion distal to the proximal end of the distal section 34. The portion distal to the proximal end, in some embodiments, may be extruded with decreasing stiffness in the distal direction, or may comprise a flattened distal section of wire or hypotube or a tapered section formed such as by grinding or stretching operations known in the art.

Disclosed herein are various offset structures or projections for centering the inner device such as a distal extension segment 34 within an outer device such as an aspiration catheter 33. Providing a centering feature between the proximal section 33 and the distal section 34 may advantageously allow only the centering feature to contacting the inside surface of the proximal section, to facilitate easy extension and retraction without binding, while maintaining centering and uniform gap spacing.

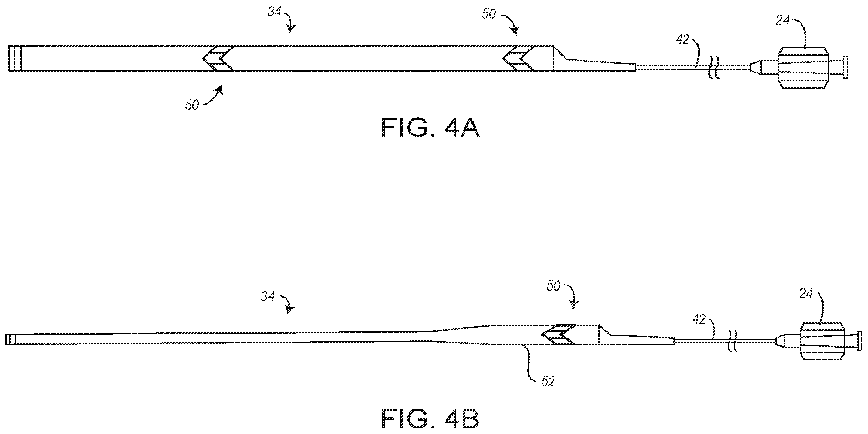

In a constant OD extension segment (Figure 4A), centering features 50 such as one or two or five or more interrupted annular bands of centering projections may be spaced axially apart along the length of the segment although typically not beyond the intended overlap 44. In a tapered or stepped OD embodiment (Figure 4B) the centering features 50 will typically be carried by a proximal, large diameter section 52.

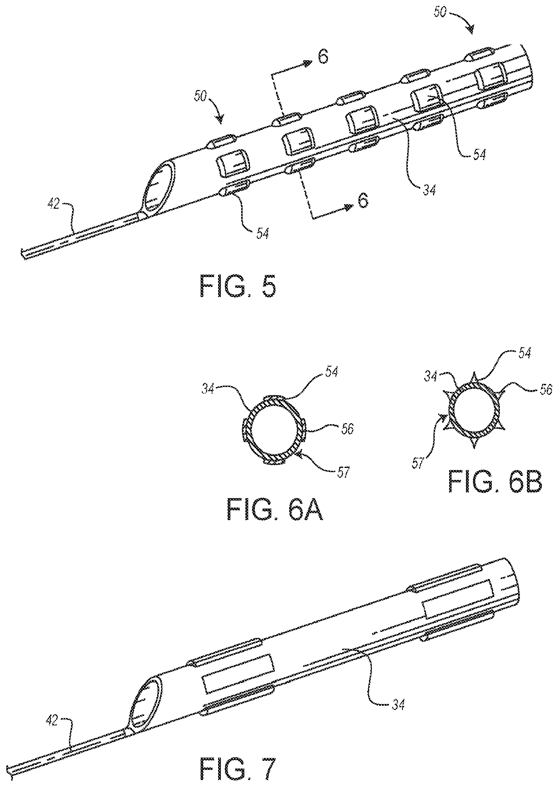

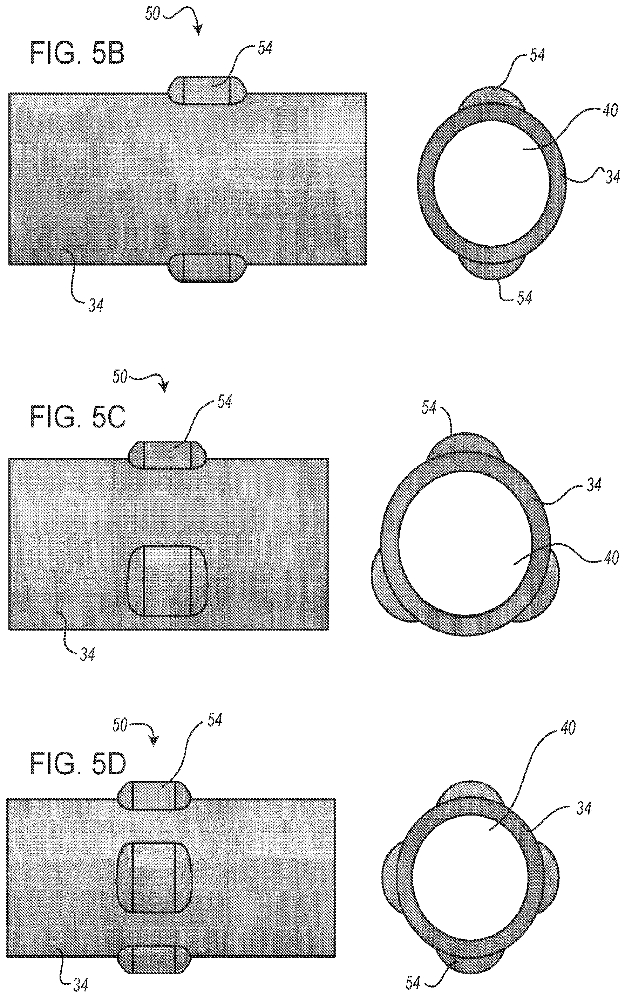

Referring to Figure 5, the centering feature 50 may comprise plurality of projections 54 extending from either the outer surface of the inner extension section, or the inner surface of the outer catheter (not illustrated). The multiple projections 54 may form at least one or two or five or ten or more ring-like annular centering features 50 around the outer surface of the inner extension segment 34 or the inner surface of the outer catheter 33.

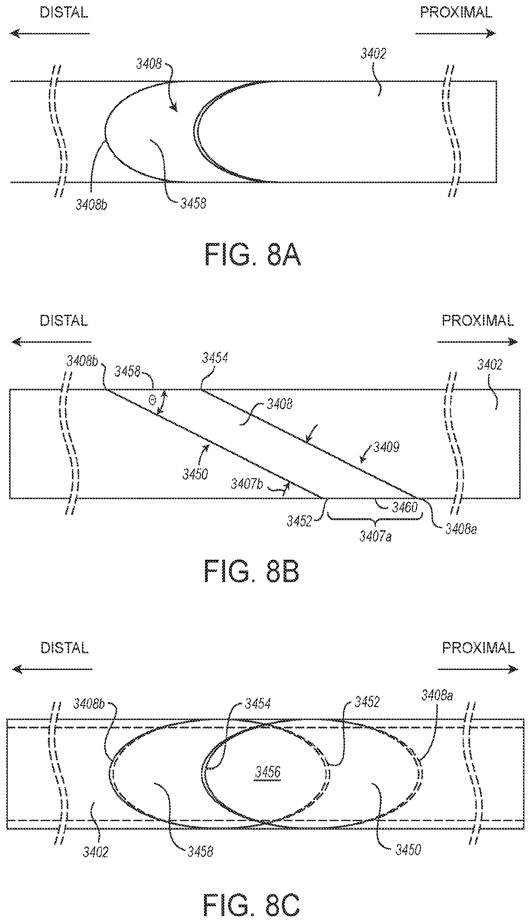

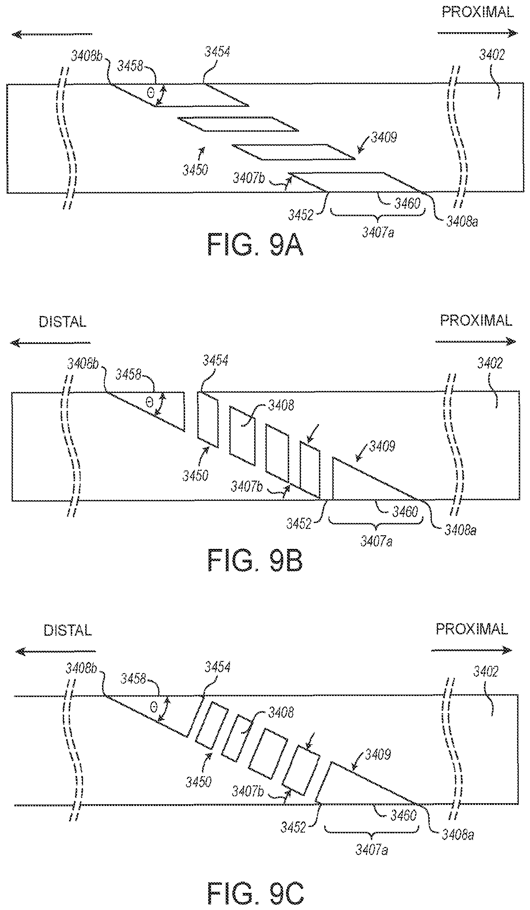

As used herein, "annular" merely suggests that the intermittent projections of the centering feature are spaced apart all the way or substantially all the way around the inner device, sufficient to achieve the centering function. However it does not require that the centering feature be continuous in a circumferential direction or reside within a single transverse plane as shown in Figures 5 and 7. Annular rings may also reside on a plane that is angled other than 90° from the longitudinal axis, such as for example shown in Figures 8 and 9.

Figure 5A illustrates five centering features 50, each having four projections 54. At least about three or five or ten or more axially spaced apart centering features 50 may be utilized, depending upon the length of the overlap 44 and desired performance of the system. Alternatively, a single centering feature may be provided, comprising a plurality of elongated axial ribs which extend axially for the entire length of the desired centering feature.

Figures 5B through 5D show a portion of a distal section 34 having a single centering feature 50. The distal section 34 may have a single centering feature 50 or may have multiple centering features 50 as has been discussed. Referring to Figure 5B, a first and a second projection 54 are provided arranged approximately 180° apart from each other around the circumference of the distal section 34. As illustrated, the projections 54 will approximately center the distal section 34 within an outer tube along a first transverse axis, however will allow movement of the distal section 34 along a second, perpendicular transverse axis, such that the sidewall of distal section 34 along the second transverse axis may be able to touch the inside surface of the outer tube. To provide centering in a two projection implementation, the circumferential length of the two projections 54 should cumulatively add up to greater than 180°, and typically at least about 190° or 200° or more.

Although most of the embodiments disclosed here in are configured to center the distal section 34 within the outer catheter, a single projection embodiment (not illustrated) such as that shown in figure 5B but with one fewer projection 54, will allow offset from the wall of the outer tube along one side of the wall of the distal section 34. This would not necessarily ensure centering of the distal section 34 within the outer catheter, however it would prevent the formation of a seal between the distal section 34 and the outer catheter. A single projection may extend circumferentially less than 360 degrees, but more than 180 degrees and achieve centering but leaving a gap to prevent creating a seal. The projection may extend at least about 220 degrees or 240 degrees but no more than 320 degrees or 290 degrees around the tubular body.

Any of the projections 54 disclosed herein can be arranged in a circumferentially asymmetrical pattern that would achieve a lateral offset of the distal section 34 in a first direction or a first and second direction to prevent formation of a seal between the distal section and the outer catheter, but not necessarily accomplish centering.

Figures 5C and 5D illustrate a three projection and a four projection centering feature 50, and will be understood to those of skill in the art in view of the disclosure herein. The implementation of Figure 5C illustrates the projections 54 as located approximately 120° apart from each other around the circumference of the distal section 34. Equidistant spacing of the projections is in some implementations desirable, but not required.

Referring to Figures 6A and 6B the projection 54 has a thickness in the radial direction between the outside diameter of the extension section 34 and a projection contact surface 56 configured for sliding contact against the surface of the wall defining the lumen of the proximal section 33. The resulting space 57 between circumferentially adjacent projections 54 forms an axially extending gap when the extension section is positioned within a proximal catheter section.

As illustrated in Figure 7, the projections may have an axial length that is longer than its width, and have a rectangular footprint on the distal section 34. That footprint may alternatively be triangular, diamond shaped or spiral around the tubular body. The footprint may be round or oval, such as in the case of a hemispherical projection.

In transverse cross section, the illustrated projections are substantially rectangular, with a curvature out of plane that matches the curvature of the OD of the distal section 34. Alternatively the transverse cross section on any of the projections can be substantially triangular, having a broadest circumferential dimension at the base with opposing longitudinal side walls tapering towards each other in a radially outwardly direction. See Figure 6B. Measured in a circumferential direction, the contact surface 56 is thus less than the base at the circumference of the underlying tube. The circumferential width of the contact surface 56 may be no more than about 75% or 50% or 25% or 10% or less of the width of the base. As the contact surface area gets smaller, the total surface contact area between the extension section and the proximal catheter is reduced.

The projection 54 may comprise an outer diameter substantially equal to the inner diameter of the proximal section 33. The projection 54 may comprise an outer diameter slightly larger than the inner diameter of the proximal section 33 but be compliant enough such that the proximal section 33 radially compresses the projection 54 while still accommodating a sliding fit.

The projection 54 may be the same material or a different material as the portion of the device (e.g., tubular body 34) from which it extends. In some embodiments, the projection 54 may comprise polyether block amide (e.g., PEBAX^{®}), polyethylene, polyurethane, Tecothane^{®}, nylon, etc. In some embodiments, the projection 54 may be formed from a softer material than the distal section 34, or at least an outer layer (e.g., an outer jacket) of the distal section 34. In some embodiments, the projection 54 may be formed from a softer material than the proximal section 33 or at least an inner layer (e.g., an inner liner) of the proximal section 33. For example, the projection 54 may be formed as part of an outer jacket of the distal section 34. In some embodiments, the projection 54 may be coextruded with the outer jacket or formed integrally with the wall such as by a grinding function.

The projections 54 may be uniformly distributed around the outer circumference of the distal section 34 (or inside surface of the proximal section 33) at a given axial position along the length of the distal section 34 within the outline of a simple transverse annular band that is 90° from the longitudinal axis of the catheter. In other embodiments, a group of projections 54 may form a centering feature 50 that inclines in an axial direction as it circumnavigates the outer circumference of the distal section 34 or the inner circumference of the proximal section 33. For example, the projection 54 may shaped as an annular circle (90° to the longitudinal axis), oval (inclined at an angle other than 90°), diamond, or other suitable shape that is inclined along the axial direction.

FIGS. 8A-8C schematically illustrate various views of an outer profile for an inclined centering feature 50 within which a plurality of projections 54 may be located. Figure 8A shows a top plan view of the elliptical, inclined profile. Figure 8B shows a side elevational view. Figure 8C shows a top plan view as in Figure 8A, also showing in phantom the portion of the outer profile on the other side of the catheter.

In the embodiment of Figure 8A, the inclined centering feature 50 may form a rounded leading distal tip 3408b from which viewing angle the tip comprises an approximately chevron-shaped profile. The view in FIG. 8C shows that the proximal tip 3408a and the distal tip 3408b are rotationally offset but otherwise symmetrical.

As shown in Figure 8D, the centering feature 50 may fit within a profile having dimensions including a width 3407a in an axial direction and an angle Θ defined relative to the longitudinal axis of the inner tubular body 3402. In some embodiments, the width 3407a of the projection 54 may be substantially constant around the circumference of the distal section 34. For example, the width 3407a may be between approximately 0.01 and 0.5 inches, or wider depending upon the nature of the projections 54.

In some embodiments, the width 3407a may be larger at and/or near the proximal trailing tip 3408a and/or the distal leading tip 3408b of the projection 54, as shown in Figure 8C. For example, the width 3407a at these points may be approximately 1.5x, 2x, 4x or larger than the width 3407b at a point where it is the smallest (e.g., halfway between the proximal point 3408a and the distal point 3408b).

In some embodiments, the angle Θ may be defined via a straight line connecting the distal tip 3408b to a point 3452 on the proximal edge of the profile on the opposite side of the extension section, relative to the longitudinal axis. In some embodiments, the angle Θ may be defined by a best fit straight line along the proximal and/or distal edge of the profile, in the case of nonlinear edges.

In some embodiments, the trailing and/or leading edge of the profile for the centering feature 50 may form an angle Θ relative to the longitudinal axis that is between about 15 and 45 degrees, 20 and 40 degrees, 20 and 30 degrees, or 30 and 40 degrees. Projection groupings having a lower angle Θ may facilitate sliding of the distal section 34 within the proximal section 33 in an axial direction.

The inclined distal edge 3450 has a first, distal transition or limit at leading point 3408b and a second, proximal limit 3452 which is axially proximally spaced from the first distal limit by at least about 2 mm; at least about 3 mm or 4 mm or more.

The inclined proximal edge 3409 extends between a distal limit 3454 and a proximal limit 3408a. As a result of this geometry, a window 3456 may be formed between the distal limit 3454 and proximal limit 3452 of the opposing edges of the profile depending upon the incline angle of the profile. See Figure 8C. A distal sliding surface 3458 is thus axially displaced from a proximal sliding surface 3460 by an amount that corresponds to the axial length of the window 3456. In the illustrated embodiment, the window 3456 has a major axis extending in the catheter longitudinal axis direction. At any point within the window, a line can be drawn transverse through the catheter without intersecting the any projections 54.

The window 3456 may have a major axis within the range of from about negative 0.5 mm (slight axial overlap of the distal sliding surface 3458 and proximal sliding surface 3460) to about 4 mm, and in some implementations at least about 1 mm or 2 mm and in some cases within the range of from about 2 mm to about 3 mm. The major axis of the window 3456 is about 2.6 mm in one implementation having projection width 3407a of about 2 mm and a catheter OD of about 0.086 inches.

An end view taken along a line perpendicular to the distal edge 3450 and or proximal edge 3409 can define an ellipse. In one implementation, the ellipse dimensions are 0.086" in the minor axis direction, which is equal to the OD of the catheter. The ellipse is about 0.2057" in the major axis direction, about 2 to 2.5 times the catheter diameter. This means the centering feature 50 inclines at about 30 degrees to the long axis of the catheter. At an incline of about 15 degrees the long axis increases to almost 4 times the catheter diameter Thus, ellipse major axis dimensions within the range of from about 1.25 to about 4 times the minor axis dimension will typically be used, often within the range of from about 1.5 to about 3X the minor axis dimension.

Preferably, the trailing edge 3454 (inside the ellipse) on one side should overlap minimally, or ideally not at all with the leading edge 3452 (inside of the ellipse) of the opposite side.

As a consequence of the angled nature of the centering feature profile 50, the overall length in the catheter axial direction from distal tip 3408b to proximal tip 3408a will often be at least about 1.5 times or two times the width 3407a. In some implementations, the overall tip to tip length may be at least about 2.5 times or 3 times the width 3407a. Multiples within the range of from about 1.5 times and about 5 times, or from about 2 times and about 3.5 times may be utilized depending upon desired performance. In one implementation having a width 3407a of about 2 mm and a catheter OD of about .086 inches, the overall axial length from tip to tip is between about 4 mm and about 8 mm, between about 6 mm and about 7 mm and optionally about 6.7 mm.

The chevron configured centering feature 50 allows for effective centering inside another tube by not requiring the other (outer) tube to have a friction fit around a full 360 degrees at the same axial location transverse plane. The contact points of the chevron allow the inner and outer tubes to be inclined slightly to each other to relieve the friction of the centering surface and at the midpoint of the chevron the outer tube can ovalize slightly while never allowing a leak path to form.

In some embodiments, the projections 54 may have uniform thickness. In some embodiments, the thickness may be between about 0.001 and 0.005 inches, 0.0015 and 0.004 inches, or 0.002 and 0.003 inches.

In some embodiments, the projections 54 within the profile may be formed from the same material as an outer layer of the distal section 34, such as Pebax^{®}. The projections may be formed by extruding the material in a similar way one or more layers of the distal section 34 are formed. The extruded material may be cut or otherwise formed into the desired profile. In some embodiments, the projection 54 may be positioned around the distal section 34 and then heated to laminate the projection 54 to the outer surface of the distal section 34, such as in a hot box. In some embodiments, the projection 54 may be adhered to the distal section 34 using an adhesive, such as a biocompatible adhesive.

In some embodiments, the projection 54 may be arranged in a spiral pattern that extends along an axial length of the distal section 34 and/or the proximal section 33 In some embodiments, the spiral may terminate at its distal end and/or proximal end in an open configuration which may allow some blood to pass through a helical passage formed by the spiral.

Figures 9A - 9C show various patterns for projections 54 arranged within the profile described in connection with Figures 8A - 8C. In figure 8A,, axially elongate projections are arranged having a longitudinal axis substantially parallel with the longitudinal axis of the catheter, and spaced apart from each other by intervening flow paths. In Figure 9B, an axis of the projection 54 such as the long axis is oriented transverse to the longitudinal axis of the catheter. In Figure 9C, an axis such as the long axis of the projection 54 is approximately perpendicular to the central plane of the inclined centering feature 50.

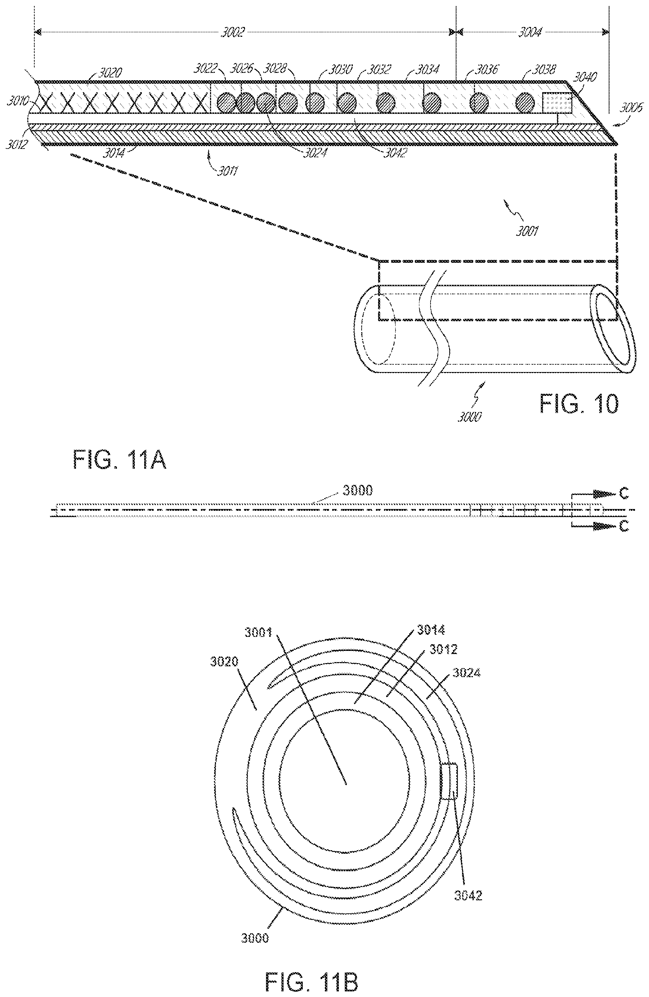

FIG. 10 illustrates a cross section through the sidewall of a distal portion of a single lumen catheter, that may be used for either or both of the proximal section 33 or distal section 34. Adjacent loops or filars of the coil 3024 may have a constant pitch throughout the length of the coil or may be closely tightly wound in a proximal zone with a distal section having looser spacing between adjacent loops. In an embodiment having a coil section 3024 with an axial length of at least between about 20% and 30% of the overall catheter length, (e.g., 28 cm coil length in a 110 cm catheter shaft 16), at least the distal 1 or 2 or 3 or 4 cm of the coil will have a spacing that is at least about 130%, and in some implementations at least about 150% or more than the spacing in the proximal coil section. In a 110 cm catheter shaft 3000 having a Nitinol coil the spacing in the proximal coil may be about 0.004 inches and in the distal section may be at least about 0.006 inches or 0.007 inches or more.

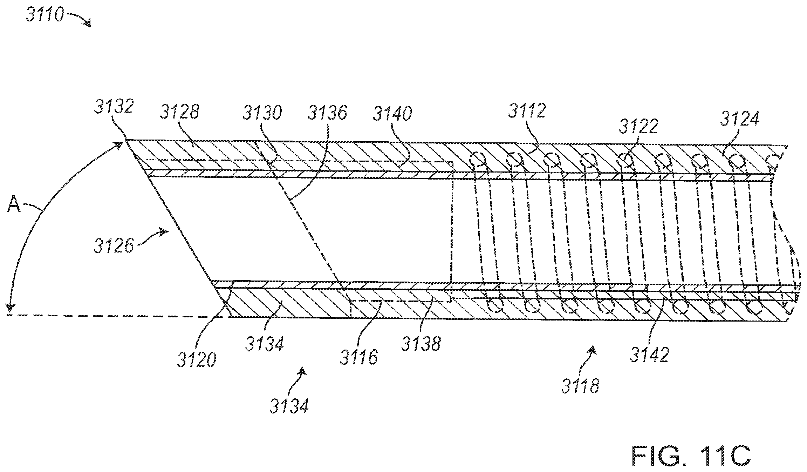

The distal end of the coil 3024 can be spaced proximally from the distal end of the inner liner 3014, for example, to provide room for an annular radiopaque marker 3040. The coil 3024 may be set back proximally from the distal end, in some embodiments, by approximately no more than 1 cm, 2 cm, or 3 cm. In one embodiment, the distal end of the catheter 10 is provided with a beveled distal surface 3006 residing on a plane having an angle of at least about 10° or 20° and in one embodiment about 30° with respect to a longitudinal axis of the catheter 10. The radiopaque marker 3040 may reside in a plane that is transverse to the longitudinal axis. Alternatively, at least the distally facing edge of the annular radiopaque marker 3040 may be an ellipse, residing on a plane which is inclined with respect to the longitudinal axis to complement the bevel angle of the distal surface 3006. Additional details are described in connection with FIG. 11C, below.

After applying the proximal braid 3010, the distal coil 3024 and the RO marker 3040 an outer jacket 3020 may be applied such as a shrink wrap tube to enclose the catheter body 16. The outer shrink-wrapped sleeve 3020 may comprise any of a variety of materials, such as polyethylene, polyurethane, polyether block amide (e.g., PEBAX^{™}), nylon or others known in the art. Sufficient heat is applied to cause the polymer to flow into and embed the proximal braid and distal coil.

In one implementation, the outer shrink wrap jacket 3020 is formed by sequentially advancing a plurality of short tubular segments 3022, 3026, 3028, 3030, 3032, 3034, 3036, 3038 concentrically over the catheter shaft subassembly, and applying heat to shrink the sections on to the catheter and provide a smooth continuous outer tubular body. The foregoing construction may extend along at least the most distal 10 cm, and preferably at least about the most distal 20 cm, 25 cm, 30 cm, 35 cm, 40 cm, or more than 40 cm of the catheter body 10. The entire length of the outer shrink wrap jacket 3020 may be formed from tubular segments and the length of the distal tubular segments (e.g., 3022, 3026, 3028, 3030, 3032, 3034, 3036, 3038) may be shorter than the one or more tubular segments forming the proximal portion of the outer shrink wrap jacket 3020 in order to provide steeper transitions in flexibility toward the distal end of the catheter 10.

The durometer of the outer wall segments may decrease in a distal direction. For example, proximal segments such as 3022 and 3026, may have a durometer of at least about 60 or 70D, with gradual decrease in durometer of successive segments in a distal direction to a durometer of no more than about 35D or 25D or lower. A 25 cm section may have at least about 3 or 5 or 7 or more segments and the catheter 10 overall may have at least about 6 or 8 or 10 or more distinct flexibility zones. The distal 1 or 2 or 4 or more segments 3036, 3038, may have a smaller OD following shrinking than the more proximal segments 3022-3034 to produce a step down in OD for the finished catheter body 16. The length of the lower OD section 3004 may be within the range of from about 3 cm to about 15 cm and in some embodiments is within the range of from about 5 cm to about 10 cm such as about 7 or 8 cm, and may be accomplished by providing the distal segments 3036, 3038 with a lower wall thickness.

Referring to FIGS. 10 and 11B, the catheter may further comprise an axial filament for increasing the tension resistance and / or influencing the bending characteristics in the distal zone. The tension support may comprise a filament and, more specifically, may comprise one or more axially extending mono strand or multi strand filaments 3042. The one or more axially extending filaments 3042 may be axially placed inside the catheter wall near the distal end of the catheter. The filament may be positioned on the convex side of the preset curve. The one or more axially extending filaments 3042 may serve as a tension support and resist elongation of the catheter wall under tension (e.g., when the catheter is being proximally retracted through tortuous vasculature).

At least one of the one or more axially extending filaments 3042 may proximally extend along the length of the catheter wall from within about 1.0 cm from the distal end of the catheter to less than about 10cm from the distal end of the catheter, less than about 20cm from the distal end of the catheter, less than about 30cm from the distal end of the catheter, less than about 40cm from the distal end of the catheter, or less than about 50cm from the distal end of the catheter.

The one or more axially extending filaments 3042 may have a length greater than or equal to about 40cm, greater than or equal to about 30cm, greater than or equal to about 20cm, greater than or equal to about 10cm, or greater than or equal to about 5cm.

At least one of the one or more axially extending filaments 3042 may extend at least about the most distal 50 cm of the length of the catheter, at least about the most distal 40 cm of the length of the catheter, at least about the most distal 30 cm or 20 cm or 10 cm of the length of the catheter.

In some implementations, the filament extends proximally from the distal end of the catheter along the length of the coil 24 and ends proximally within about 5 cm or 2 cm or less either side of the transition 3011 between the coil 3024 and the braid 3010. The filament may end at the transition 3011, without overlapping with the braid 3010.

In another embodiment, the most distal portion of the catheter 10 may comprise a durometer of less than approximately 35D (e.g., 25D) to form a highly flexible distal portion of the catheter and have a length between approximately 25 cm and approximately 35 cm. The distal portion may comprise one or more tubular segments of the same durometer (e.g., segment 3038). A series of proximally adjacent tubular segments may form a transition region between a proximal stiffer portion of the catheter 3000 and the distal highly flexible portion of the catheter. The series of tubular segments forming the transition region may have the same or substantially similar lengths, such as approximately 1 cm.

The relatively short length of each of the series of tubular segments may provide a steep drop in durometer over the transition region. For example, the transition region may have a proximal tubular segment 3036 (proximally adjacent the distal portion) having a durometer of approximately 35D. An adjacent proximal segment 3034 may have a durometer of approximately 55D. An adjacent proximal segment 3032 may have a durometer of approximately 63D. An adjacent proximal segment 3030 may have a durometer of approximately 72D.

More proximal segments may comprise a durometer or durometers greater than approximately 72D and may extend to the proximal end of the catheter or extension catheter segment. For instance, an extension catheter segment may comprise a proximal portion greater than approximately 72D between about 1 cm and about 3 cm. In some embodiments, the proximal portion may be about 2 cm long. In some embodiments, the most distal segments (e.g., 3038-3030) may comprise PEBAX^{™} and more proximal segments may comprise a generally stiffer material, such as Vestamid^{®}.

The inner diameter of the catheter 10 may be between approximately 0.06 and 0.08 inches, between approximately 0.065 and 0.075 inches, or between 0.068 and 0.073 inches. In some embodiments, the inner diameter is approximately 0.071 inches.

In some embodiments, the distal most portion may taper to a decreased inner diameter as described elsewhere herein. The taper may occur approximately between the distal highly flexible portion and the transition region (e.g., over the most proximal portion of the distal highly flexible portion). The taper may be relatively gradual (e.g., occurring over approximately 10 or more cm) or may be relatively steep (e.g., occurring over less than approximately 5 cm). The inner diameter may taper to an inner diameter between about 0.03 and 0.06 inches. For example, the inner diameter may be about 0.035 inches, about 0.045 inches, or about 0.055 inches at the distal end of the catheter 3000. In some embodiments, the inner diameter may remain constant, at least over the catheter extension segment.

In some embodiments, the coil 3024 may extend proximally from a distal end of the catheter 10 along the highly flexible distal portion ending at the distal end of the transition region. In other embodiments, the coil 3024 may extend from a distal end of the catheter to the proximal end of the transition region, to a point along the transition region, or proximally beyond the transition region. In other embodiments, the coil 3024 may extend the entire length of the catheter 10 or catheter extension segment as described elsewhere herein. The braid 3010, when present, may extend from the proximal end of the coil 3024 to the proximal end of the catheter 10.

The one or more axially extending filaments 3042 may be placed near or radially outside the tie layer 3012 or the inner liner 3014. The one or more axially extending filaments 3042 may be placed near or radially inside the braid 3010 and/or the coil 3024. The one or more axially extending filaments 3042 may be carried between the inner liner 3014 and the helical coil 3024, and may be secured to the inner liner or other underlying surface by an adhesive prior to addition of the next outer adjacent layer such as the coil.

When more than one axially extending filaments 3042 are placed in the catheter wall, the axially extending filaments 3042 may be placed in a radially symmetrical manner. For example, the angle between the two axially extending filaments 3042 with respect to the radial center of the catheter may be about 180 degree. Alternatively, depending on desired clinical performances (e.g., flexibility, trackability), the axially extending filaments 3042 may be placed in a radially asymmetrical manner. The angle between any two axially extending filaments 3042 with respect to the radial center of the catheter may be less than about 180 degree, less than or equal to about 165 degree, less than or equal to about 150 degree, less than or equal to about 135 degree, less than or equal to about 120 degree, less than or equal to about 105 degree, less than or equal to about 90 degree, less than or equal to about 75 degree, less than or equal to about 60 degree, less than or equal to about 45 degree, less than or equal to about 30 degree, less than or equal to about 15 degree, less than or equal to about 10 degree, or less than or equal to about 5 degree.

The one or more axially extending filaments 3042 may be made of materials such as Vectran, Kevlar, Polyester, Meta-Para-Aramide, or any combinations thereof. At least one of the one or more axially extending filaments 3042 may comprise a single fiber or a multi-fiber bundle, and the fiber or bundle may have a round or rectangular cross section. The terms fiber or filament do not convey composition, and they may comprise any of a variety of high tensile strength polymers, metals or alloys depending upon design considerations such as the desired tensile failure limit and wall thickness. The cross-sectional dimension of the one or more axially extending filaments 3042, as measured in the radial direction, may be no more than about 2%, 5%, 8%, 15%, or 20% of that of the catheter 10.

The cross-sectional dimension of the one or more axially extending filaments 3042, as measured in the radial direction, may be no more than about 0.001 inches, about 0.002 inches, about 0.004 inches, about 0.006 inches, about 0.008 inches, or about 0.015 inches.

The one or more axially extending filaments 3042 may increase the tensile strength of the distal zone of the catheter before failure under tension to at least about 1 pound, at least about 2 pounds, at least about 3 pounds, at least about 4 pounds, at least about 5 pounds, at least about 6 pounds, at least about 7 pounds, at least about 8 pounds, or at least about 10 pounds or more.

Any of the catheters disclosed herein, whether or not an axial filament is included, may be provided with an angled distal tip. Referring to Figure 11C, distal catheter tip 3110 comprises a tubular body 3112 which includes an advance segment 3114, a marker band 3116 and a proximal segment 3118. An inner tubular liner 3120 may extend throughout the length of the distal catheter tip 3110, and may comprise dip coated PTFE.

A reinforcing element 3122 such as a braid or spring coil is embedded in an outer polymer jacket 3124 which may extend the entire length of the distal catheter tip 3110.

The advance segment 3114 terminates distally in an angled face 3126, to provide a leading side wall portion 3128 having a length measured between the distal end 3130 of the marker band 3116 and a distal tip 3132. A trailing side wall portion 3134 of the advance segment 3114, has an axial length in the illustrated embodiment of approximately equal to the axial length of the leading side wall portion 3128 as measured at approximately 180 degrees around the catheter from the leading side wall portion 3128. The leading side wall portion 3128 may have an axial length within the range of from about 0.1 mm to about 5 mm and generally within the range of from about 1 to 3 mm. The trailing side wall portion 3134 may be at least about 0.1 or 0.5 or 1 mm or 2 mm or more shorter than the axial length of the leading side wall portion 3128, depending upon the desired performance.

The angled face 3126 inclines at an angle A within the range of from about 45 degrees to about 80 degrees from the longitudinal axis of the catheter. For certain implementations, the angle is within the range of from about 55 degrees to about 65 degrees or within the range of from about 55 degrees to about 65 degrees from the longitudinal axis of the catheter. In one implementation the angle A is about 60 degrees. One consequence of an angle A of less than 90 degrees is an elongation of a major axis of the area of the distal port which increases the surface area of the port and may enhance clot aspiration or retention. Compared to the surface area of the circular port (angle A is 90 degrees), the area of the angled port is generally at least about 105%, and no more than about 130%, in some implementations within the range of from about 110% and about 125% and in one example is about 115%.

In the illustrated embodiment, the axial length of the advance segment is substantially constant around the circumference of the catheter, so that the angled face 3126 is approximately parallel to the distal surface 3136 of the marker band 3116. The marker band 3116 has a proximal surface approximately transverse to the longitudinal axis of the catheter, producing a marker band 3116 having a right trapezoid configuration in side elevational view. A short sidewall 3138 is rotationally aligned with the trailing side wall portion 3134, and has an axial length within the range of from about 0.2 mm to about 4 mm, and typically from about 0.5 mm to about 2 mm. An opposing long sidewall 3140 is rotationally aligned with the leading side wall portion 3128. Long sidewall 3140 of the marker band 3116 is generally at least about 10% or 20% longer than short sidewall 3138 and may be at least about 50% or 70% or 90% or more longer than short sidewall 3138, depending upon desired performance. Generally the long sidewall 3140 will have a length of at least about 0.5 mm or 1 mm and less than about 5 mm or 4 mm.

The marker band may have at least one and optionally two or three or more axially extending slits throughout its length to facilitate manufacturing and / or enable radial expansion. The slit may be located on the short sidewall 3138 or the long sidewall 3140 or in between, depending upon desired bending characteristics. The marker band may comprise any of a variety of radiopaque materials, such as a platinum / iridium alloy, with a wall thickness preferably no more than about 0.003 inches and in one implementation is about 0.001 inches. In one implementation, at least one axial slit is aligned with the convex side of the preset curve, and the filament extends distally beyond the proximal face of the marker and into the axial slit.

The marker band zone of the assembled catheter will have a relatively high bending stiffness and high crush strength, such as at least about 50% or at least about 100% less than proximal segment 18 but generally no more than about 200% less than proximal segment 3118. The high crush strength may provide radial support to the adjacent advance segment 3114 and particularly to the leading side wall portion 3128, to facilitate the functioning of distal tip 3132 as an atraumatic bumper during transluminal advance and to resist collapse under vacuum. The proximal segment 3118 preferably has a lower bending stiffness than the marker band zone, and the advance segment 3114 preferably has even a lower bending stiffness and crush strength than the proximal segment 3118.

The advance segment 3114 may comprise a distal extension of the outer jacket 3124 and optionally the inner liner 3120, without other internal supporting structures distally of the marker band 3116. Outer jacket may comprise extruded Tecothane. The advance segment 3114 may have a bending stiffness and radial crush stiffness that is no more than about 50%, and in some implementations no more than about 25% or 15% or 5% or less than the corresponding value for the proximal segment 3118.

A support fiber 3142 as has been discussed elsewhere herein extends through at least a distal portion of the length of the proximal segment 3118. As illustrated, the support fiber 3142 may terminate distally at a proximal surface of the marker band 3116 and extend axially radially outwardly of the tubular liner 3120 and radially inwardly from the support coil 3122. Alternatively, the marker band may be provided with at least one or two axially extending slots, and the fiber can extend into the slot, thus axially overlapping with the marker band. Fiber 3142 may extend substantially parallel to the longitudinal axis, or may be inclined into a mild spiral having no more than 10 or 7 or 3 or 1 or less complete revolutions around the catheter along the length of the spiral. The fiber may comprise a high tensile strength material such as a multifilament yarn spun from liquid crystal polymer such as a Vectran multifilament LCP fiber.

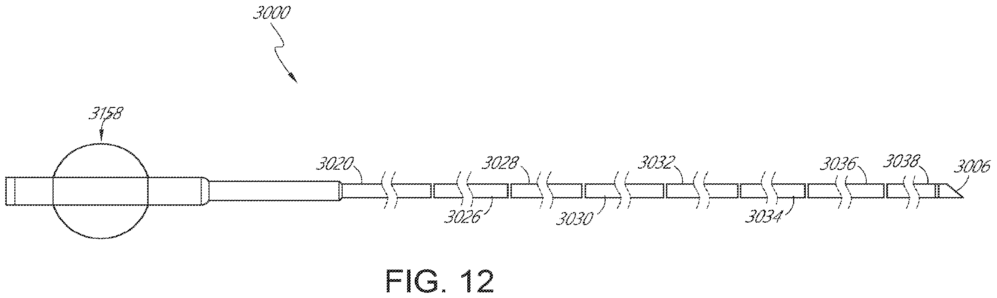

Referring to FIG. 12, there is illustrated one example of an outer jacket segment stacking pattern for a progressive flexibility catheter of the type discussed above. A distal segment 3038 may have a length within the range of about 1 - 3 cm, and a durometer of less than about 35D or 30D. An adjacent proximal segment 3036 may have a length within the range of about 4 - 6 cm, and a durometer of less than about 35D or 30D. An adjacent proximal segment 3034 may have a length within the range of about 4 - 6 cm, and a durometer of about 35D or less. An adjacent proximal segment 3032 may have a length within the range of about 1 - 3 cm, and a durometer within the range of from about 35D to about 45D (e.g., 40D). An adjacent proximal segment 3030 may have a length within the range of about 1 - 3 cm, and a durometer within the range of from about 50D to about 60D (e.g., about 55D). An adjacent proximal segment 3028 may have a length within the range of about 1 - 3 cm, and a durometer within the range of from about 35D to about 50D to about 60D (e.g., about 55D). An adjacent proximal segment 3026 may have a length within the range of about 1 - 3 cm, and a durometer of at least about 60D and typically less than about 75D.

More proximal segments may have a durometer of at least about 65D or 70D. The distal most two or three segments may comprise a material such as Tecothane, and more proximal segments may comprise PEBAX or other catheter jacket materials known in the art. At least three or five or seven or nine or more discrete segments may be utilized, having a change in durometer between highest and lowest along the length of the catheter shaft of at least about 10D, preferably at least about 20D and in some implementations at least about 30D or 40D or more.

Although the present invention has been described in terms of certain preferred embodiments, it may be incorporated into other embodiments by persons of skill in the art in view of the disclosure herein. The scope of the invention is therefore not intended to be limited by the specific embodiments disclosed herein, but is intended to be defined by the full scope of the following claims.

## Claims

1. An extendable neurovascular catheter, comprising:
an outer catheter (33) having a proximal end, a distal end, a sidewall defining a central lumen, and a longitudinal axis;
an inner extension segment (34) having a proximal end (36) and a distal end (38) and extendable through the central lumen such that the distal end (38) of the inner extension segment (34) is configured to extend beyond the distal end of the outer catheter;
an annular gap between an outer surface of the inner extension segment (34) and an inner surface of the central lumen; and
a plurality of projections (54) positioned between the inner extension segment (34) and the outer catheter (33) and configured to center at least a portion of a length of the inner extension segment (34) within the central lumen, and to maintain a preset distance between the inner extension segment (34) and the sidewall;
**characterized in that** the plurality of projections (54) are spaced apart from each other by intervening flow paths.

2. The extendable neurovascular catheter of claim 1, comprising at least three projections (54).

3. The extendable neurovascular catheter of claim 1 wherein the plurality of projections (54) are carried on an outside surface of the inner extension segment (34).

4. The extendable neurovascular catheter of claim 1, further comprising an axially movable control wire (42) extending proximally through the outer catheter (33) from the inner extension segment (34).

5. The extendable neurovascular catheter of claim 1, comprising at least one centering feature (50) made from the plurality of projections (54) arranged in a pattern, the at least one centering feature (50) extending circumferentially around the inner extension segment (34).

6. The extendable neurovascular catheter of claim 5, wherein the at least one centering feature (50) is inclined at a non normal angle with respect to the longitudinal axis.

7. The extendable neurovascular catheter of claim 3, wherein the plurality of projections (54) are spaced axially apart along the length of the inner extension segment (34).

8. The extendable neurovascular catheter of claim 1, wherein the plurality of projections (54) extend from an inner surface of the outer catheter (33).

9. The extendable neurovascular catheter of claim 8, wherein the plurality of projections (54) are spaced axially apart along a length of the outer catheter (33).

10. The extendable neurovascular catheter of claim 1, comprising at least one centering feature (50) made from one or more interrupted annular bands of projections (54) of the plurality of projections (54) spaced axially apart.

11. The extendable neurovascular catheter of claim 1, wherein a longitudinal axis of each projection (54) of the plurality of projections (54) is substantially parallel with the longitudinal axis of the outer catheter (33).

12. The extendable neurovascular catheter of claim 1, wherein a long axis of each projection of the plurality of projections (54) is transverse to the longitudinal axis of the outer catheter (33).

## Patentansprüche

1. Ein verlängerbarer neurovaskulärer Katheter, umfassend:
einen äußeren Katheter (33) mit einem proximalen Ende, einem distalen Ende, einer Seitenwand, die ein zentrales Lumen definiert, und einer Längsachse;
ein inneres Verlängerungssegment (34) mit einem proximalen Ende (36) und einem distalen Ende (38) und das durch das zentrale Lumen verlängerbar ist, derart, dass das distale Ende (38) des inneren Verlängerungssegments (34) dazu eingerichtet ist, sich über das distale Ende des äußeren Katheters hinaus zu erstrecken;
einen ringförmigen Spalt zwischen einer Außenoberfläche des inneren Verlängerungssegments (34) und einer Innenoberfläche des zentralen Lumens; und
eine Mehrzahl von Vorsprüngen (54), die zwischen dem inneren Verlängerungssegment (34) und dem äußeren Katheter (33) positioniert sind und dazu eingerichtet sind, zumindest einen Abschnitt einer Länge des inneren Verlängerungssegments (34) innerhalb des zentralen Lumens zu zentrieren und einen voreingestellten Abstand zwischen dem inneren Verlängerungssegment (34) und der Seitenwand zu erhalten;
**dadurch gekennzeichnet, dass** die Mehrzahl von Vorsprüngen (54) durch dazwischenliegende Strömungspfade voneinander beabstandet sind.

2. Der verlängerbare neurovaskuläre Katheter nach Anspruch 1, umfassend mindestens drei Vorsprünge (54).

3. Der verlängerbare neurovaskuläre Katheter nach Anspruch 1, wobei die Mehrzahl von Vorsprüngen (54) von einer Außenseite des inneren Verlängerungssegments (34) getragen sind.

4. Der verlängerbare neurovaskuläre Katheter nach Anspruch 1, ferner umfassend einen axial beweglichen Steuerdraht (42), der sich von dem inneren Verlängerungssegment (34) proximal durch den äußeren Katheter (33) erstreckt.

5. Der verlängerbare neurovaskuläre Katheter nach Anspruch 1, umfassend mindestens ein Zentrierelement (50), das aus der Mehrzahl von Vorsprüngen (54) besteht, die in einem Muster angeordnet sind, wobei das mindestens eine Zentrierelement (50) sich umlaufend um das innere Verlängerungssegment (34) erstreckt.

6. Der verlängerbare neurovaskuläre Katheter nach Anspruch 5, wobei das mindestens eine Zentrierelement (50) in einem nicht normalen Winkel bezüglich der Längsachse geneigt ist.

7. Der verlängerbare neurovaskuläre Katheter nach Anspruch 3, wobei die Mehrzahl von Vorsprüngen (54) axial entlang der Länge des inneren Verlängerungssegments (34) beabstandet sind.

8. Der verlängerbare neurovaskuläre Katheter nach Anspruch 1, wobei die Mehrzahl von Vorsprüngen (54) sich von einer Innenoberfläche des äußeren Katheters (33) erstrecken.

9. Der verlängerbare neurovaskuläre Katheter nach Anspruch 8, wobei die Mehrzahl von Vorsprüngen (54) axial entlang einer Länge des äußeren Katheters (33) beabstandet sind.

10. Der verlängerbare neurovaskuläre Katheter nach Anspruch 1, umfassend mindestens ein Zentrierelement (50), das aus einer oder mehreren unterbrochenen ringförmigen Bändern von Vorsprüngen (54) der Mehrzahl von Vorsprüngen (54) besteht, die axial beabstandet sind.

11. Der verlängerbare neurovaskuläre Katheter nach Anspruch 1, wobei eine Längsachse jedes Vorsprungs (54) der Mehrzahl von Vorsprüngen (54) im Wesentlichen parallel zu der Längsachse des äußeren Katheters (33) ist.

12. Der verlängerbare neurovaskuläre Katheter nach Anspruch 1, wobei eine lange Achse jedes Vorsprungs der Mehrzahl von Vorsprüngen (54) quer zu der Längsachse des äußeren Katheters (33) ist.

## Revendications

1. Un cathéter neurovasculaire extensible, comprenant :
un cathéter externe (33) ayant une extrémité proximale, une extrémité distale, une paroi latérale définissant une lumière centrale, et un axe longitudinal ;
un segment d'extension interne (34) ayant une extrémité proximale (36) et une extrémité distale (38) et extensible à travers la lumière centrale de telle sorte que l'extrémité distale (38) du segment d'extension interne (34) soit configurée pour s'étendre au-delà de l'extrémité distale du cathéter externe ;
un espace annulaire entre une surface extérieure du segment d'extension interne (34) et une surface interne de la lumière centrale ; et
une pluralité de saillies (54) positionnées entre le segment d'extension interne (34) et le cathéter externe (33) et configurées pour centrer au moins une partie de la longueur du segment d'extension interne (34) à l'intérieur de la lumière centrale, et pour maintenir une distance prédéfinie entre le segment d'extension interne (34) et la paroi latérale ;
**caractérisé en ce que** la pluralité de saillies (54) sont espacées les unes des autres par des voies d'écoulement intermédiaires.

2. Le cathéter neurovasculaire extensible selon la revendication 1, comprenant au moins trois saillies (54).

3. Le cathéter neurovasculaire extensible selon la revendication 1, dans lequel la pluralité de saillies (54) sont portées sur une surface extérieure du segment d'extension interne (34).

4. Le cathéter neurovasculaire extensible selon la revendication 1, comprenant en outre un fil de commande (42) mobile axialement s'étendant de manière proximale à travers le cathéter externe (33) à partir du segment d'extension interne (34).

5. Le cathéter neurovasculaire extensible selon la revendication 1, comprenant au moins un élément de centrage (50) constitué par la pluralité de saillies (54) disposées selon un motif, ledit au moins un élément de centrage (50) s'étendant circonférentiellement autour du segment d'extension interne (34).

6. Le cathéter neurovasculaire extensible selon la revendication 5, dans lequel ledit au moins un élément de centrage (50) est incliné selon un angle non normal par rapport à l'axe longitudinal.

7. Le cathéter neurovasculaire extensible selon la revendication 3, dans lequel la pluralité de saillies (54) sont espacées axialement sur la longueur du segment d'extension interne (34).

8. Le cathéter neurovasculaire extensible selon la revendication 1, dans lequel la pluralité de saillies (54) s'étendent à partir d'une surface interne du cathéter externe (33).

9. Le cathéter neurovasculaire extensible selon la revendication 8, dans lequel la pluralité de saillies (54) sont espacées axialement sur une longueur du cathéter externe (33).

10. Le cathéter neurovasculaire extensible selon la revendication 1, comprenant au moins un élément de centrage (50) constitué d'une ou plusieurs bandes annulaires discontinues de saillies (54) de la pluralité de saillies (54) espacées axialement.

11. Le cathéter neurovasculaire extensible selon la revendication 1, dans lequel l'axe longitudinal de chaque saillie (54) de la pluralité de saillies (54) est sensiblement parallèle à l'axe longitudinal du cathéter externe (33).

12. Le cathéter neurovasculaire extensible selon la revendication 1, dans lequel un axe long de chaque saillie de la pluralité de saillies (54) est transversal à l'axe longitudinal du cathéter externe (33).
